(19) Europäisches Patentamt
European Patent Office
Office européen des brevets

(11) EP 2 450 334 B1

(12) EUROPÄISCHE PATENTSCHRIFT

(45) Veröffentlichungstag und Bekanntmachung des Hinweises auf die Patenterteilung:
**11.06.2014 Patentblatt 2014/24**

(51) Int Cl.:
***C07C 29/84*** (2006.01) ***C07C 35/08*** (2006.01)
***C07C 35/12*** (2006.01) ***B01D 3/40*** (2006.01)

(21) Anmeldenummer: **10013850.2**

(22) Anmeldetag: **21.10.2010**

(54) **Verfahren zur Trennung von isomeren Mentholverbindungen**

Method for separating isomeric menthol compounds

Procédé de séparation de composés de menthol isomères

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.05.2012 Patentblatt 2012/19**

(73) Patentinhaber: **LANXESS Deutschland GmbH
50569 Köln (DE)**

(72) Erfinder:
- **Brink, Reinhold
45721 Haltern (DE)**
- **Heuer, Lutz
41452 Dormagen (DE)**
- **Kuhlmann, Sven
50733 Köln (DE)**
- **Mechelhoff, Martin
50733 Köln (DE)**
- **Pfohl, Oliver
22587 Hamburg (DE)**

(56) Entgegenhaltungen:
**DE-A1- 10 136 614 DE-A1- 10 154 052
US-A- 4 874 473**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 450 334 B1

**Beschreibung**

Die Erfindung betrifft ein Verfahren zur rektifikativen Trennung von Stoffgemischen enthaltend Diastereomere des 2-Isopropyl-5-methylcyclohexanols unter Einsatz von ionischen Flüssigkeiten als Extraktionsmittel.

Die Rektifikation ist ein in der Technik verbreitet angewandte Methode um Stoffgemische voneinander zu trennen. Der erforderliche Aufwand für die Trennung eines Stoffgemisches bei der Rektifikation wird durch den sogenannten Trennfaktor $\alpha$, dem Verhältnis der Verteilungskoeffizienten der beteiligten Komponenten wiedergegeben. Je höher der Abstand des Trennfaktors vom Wert eins ist, desto wirtschaftlicher lässt sich in der Regel das Stoffgemisch trennen.

Viele Stoffgemische, deren Komponenten nur geringe Siedepunktsunterschiede aufweisen oder gar Azeotrope bilden, sind durch konventionelle Rektifikation nur schwer bzw. gar nicht trennbar. Dies trifft beispielsweise auf die Trennung von Diastereomeren des 2-Isopropyl-5-methylcyclohexanols (d,1-Menthol, d,1-Neomenthol, d,1-Isomenthol und d,1-Neoisomenthol) aus Stoffgemischen enthaltend zumindest zwei zueinander diastereomere Verbindungen des 2-Isopropyl-5-methylcyclohexanols zu, wie sie typischerweise bei der Hydrierung von Thymol oder nachfolgenden Aufarbeitungsschritten entstehen. Insbesondere die Trennung der Diastereomeren Isomenthol und Menthol ist aufgrund der geringen relativen Flüchtigkeit der beiden Verbindungen zueinander nur unzureichend und unter hohem Energieeinsatz zu leisten.

In solchen Fällen wird dem zu trennenden Stoffgemisch typischerweise ein selektiver Zusatzstoff, ein sogenannter Entrainer zugesetzt, der über selektive Wechselwirkungen mit den Komponenten des Stoffgemisches das Dampf-Flüssig-Phasengleichgewicht in der Art beeinflusst, dass bessere Trennfaktoren erreicht werden, als ohne Zusatz der Entrainer.

Typischerweise sinkt dadurch der Energie- bzw. Kostenaufwand für die Trennung eingesetzten Stoffgemisches. In jüngster Zeit sind auch ionische Flüssigkeiten als Entrainer Gegenstand von intensiven Untersuchungen geworden (siehe auch DE 10136614 A, DE 10154052 A und Chem. Ing. Tech. 2003, 75, 1148-1149).

Aus der Literatur ist eine Vielzahl von Entrainem für die Trennung von Stereoisomeren Verbindungen bekannt.

So beschreibt US 4 874 473 A die Verwendung von Succinsäurediamid sowie Succinsäure-, Glutarsäure- und Malonsäuredinitril zur Trennung von Gemischen aus Isomenthol und Menthol.

Vor dem Hintergrund des vorstehend genannten Standes der Technik bestand die Aufgabe, ein Verfahren bereitzustellen, mit dem die Diastereomeren des 2-Isopropyl-5-methylcyclohexanols besonders effizient getrennt werden können.

Gegenstand der Erfindung ist nun ein Verfahren zur Trennung von Diastereomeren des 2-Isopropyl-5-methylcyclohexanols aus Stoffgemischen enthaltend zumindest zwei zueinander diastereomere Verbindungen des 2-Isopropyl-5-methylcyclohexanols mittels extraktiver Rektifikation, das dadurch gekennzeichnet ist, dass die Rektifikation in Gegenwart einer oder mehrerer ionischer Flüssigkeiten durchgeführt wird.

Unter ionischen Flüssigkeiten sind im Sinne der Erfindung Verbindungen zu verstehen, die mindestens ein Kation oder eine kationische Gruppe und mindestens ein Anion oder eine anionische Gruppe aufweisen, insgesamt jedoch ladungsneutral sind, und einen Schmelzpunkt unter 200°C aufweisen.

Bevorzugte ionische Flüssigkeiten sind solche, die ein organisches Kation aufweisen.

Besonders bevorzugte ionische Flüssigkeiten sind solche der Formel (I)

$$[K^+]\,[A^-] \qquad (I)$$

in der

$[K^+]$ für ein Kation steht, das ausgewählt ist aus der Gruppe der Formeln (IIa) bis (IIe)

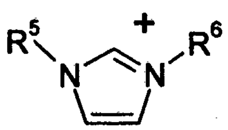

(IIa)

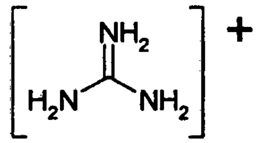

(IIb)

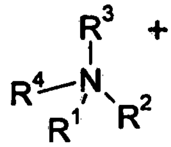

(IIc)

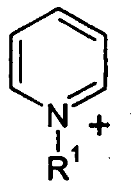 (IId) 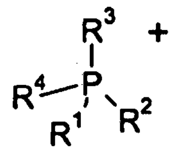 (IIe)

und
$[A^-]$ für ein Anion steht, das ausgewählt ist aus der Gruppe:

Hexafluorophosphat, Tetrafluoroborat, Chlorid, Bromid, Iodid, Bis(trifluormethylsulfonyl)amid und solchen der Formeln (IIIa), (IIIb) und (IIIc) (IIIa) $R^7$-$SO_3^-$ (IIIb) $^-O(P{=}O)(OR^3)(OR^4)$ (IIIc) $^-O(P{=}O)(R^3)(OR^4)$
wobei in den Formeln (IIa) bis (IIe) sowie den Formeln (IIIa) bis (IIIc)

- die Reste $R^1$, $R^2$, $R^3$ und $R^4$ jeweils völlig unabhängig voneinander für Wasserstoff, $C_1$-$C_{12}$-Alkyl, $C_1$-$C_{12}$-Fluoralkyl, $C_2$-$C_{12}$-Alkylen oder $C_1$-$C_{12}$-Hydroxyalkyl stehen
  wobei $C_1$-$C_{12}$-Alkyl, beziehungsweise $C_2$-$C_{12}$-Alkylen, beziehungsweise $C_2$-$C_{12}$-Alkenylen jeweils unabhängig einem geradkettigen, ganz oder teilweise cyclischen, verzweigten oder unverzweigten Alkyl-, beziehungsweise Alkylen-, beziehungsweise Alkenylen-Rest mit der angegebenen Zahl an Kohlenstoffatomen entspricht und wobei die genannten Reste nicht, einfach oder mehrfach, bevorzugt nicht oder einfach weiterhin durch $C_1$-$C_4$-Alkoxyreste oder Halogene substituiert sein können
  oder
  zwei Reste zusammen für $C_2$-$C_{12}$-Alkenylen stehen
  wobei für die Formeln (IIc) und (IIe) die Maßgabe gilt dass maximal zwei der Reste $R^1$, $R^2$, $R^3$ und $R^4$ für Wasserstoff stehen
  und

- die Reste $R^5$ und $R^6$ jeweils völlig unabhängig voneinander für Wasserstoff, Hydroxy, $C_1$-$C_{12}$-Alkyl, $C_1$-$C_{12}$-Fluoralkyl oder $C_1$-$C_{12}$-Hydroxyalkyl stehen
  und

- der Rest $R^7$ für einen Phenyl-Rest steht, der nicht, einfach oder mehrfach durch Reste substituiert ist die ausgewählt sind aus der Gruppe $C_1$-$C_{12}$-Alkyl, $C_1$-$C_{12}$-Fluoralkyl, Fluor, Chlor, Brom, Hydroxy oder $C_1$-$C_{12}$-Hydroxyalkyl.

Ganz besonders bevorzugte Ionische Flüssigkeiten sind solche der Formel (I) in denen $[K^+]$ für ein Kation der Formel (IIa) oder (IIb) steht und $[A^-]$ für Hexafluorophosphat oder Bis(trifluormethylsulfonyl)amid steht.
Noch weiter bevorzugte Verbindungen der Formel (I) sind:

1,3-Dihydroxyimidazolium-hexafluorophosphat,Guanidinium-bis(trifluoromethylsulfonyl)-amid, 2-Hydroxyethylammonium-bis(trifluoromethylsulfonyl)amid, Imidazolium-bis(trifluoromethylsulfonyl)amid, Butylammonium-triflat, Trimethylammonium-methansulfonat, 1,3-Dihydroxyimidazolium-bis(trifluoromethylsulfonyl)amid, N,N-Dimethylethanolammonium-bis(trifluoromethylsulfonyl)amid, 1,3-Dihydroxy-2-methyl-imidazolium-bis(trifluoromethylsulfonyl)amid, 1-Butylimidazolium-hexafluorophosphat und Guanidinium-tris(pentafluoroethyl)trifluorophosphat.

Ganz besonders bevorzugte Verbindungen der Formel (I) sind 1,3-Dihydroxyimidazolium-hexafluorophosphat, Guanidinium-bis(trifluoromethylsulfonyl)amid, Imidazolium-bis(trifluoromethylsulfonyl)amid und 1,3-Dihydroxyimidazolium-bis(trifluoromethyl-sulfonyl)amid.
$C_1$-$C_{12}$-Alkyl beziehungsweise $C_2$-$C_{12}$-Alkylen beziehungsweise $C_2$-$C_{12}$-Alkenylen bedeutet jeweils unabhängig einen geradkettigen, ganz oder teilweise cyclischen, verzweigten oder unverzweigten Alkyl beziehungsweise Alkylen-beziehungsweise Alkenylen-Rest mit der angegebenen Zahl an Kohlenstoffatomen, wobei die genannten Reste nicht, einfach oder mehrfach, bevorzgt nicht oder einfach weiterhin durch $C_1$-$C_4$-Alkoxyreste oder Halogene substituiert sein können.
$C_1$-$C_{12}$-Alkyl steht beispielsweise für Methyl, Ethyl, 2-Methoxyethyl, n-Propyl, iso-Propyl, n-Butyl, sec.-Butyl und tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl, n-Octyl, Adamantyl, die isomeren Menthyle, n-Nonyl, n-Decyl oder n-Dodecyl.

$C_1$-$C_4$-Alkoxy steht beispielsweise für Methoxy, Ethoxy, 2-Methoxyethoxy, n-Propoxy, iso-Propoxy, n-Butoxy, sec.-Butoxy oder tert.-Butoxy.

$C_1$-$C_{12}$-Fluoralkyl bedeutet jeweils unabhängig einen geradkettigen, ganz oder teilweise cyclischen, verzweigten oder unverzweigten Alkylrest der einfach oder mehrfach, vorzugsweise vollständig durch Fluoratome substituiert ist.

$C_1$-$C_{12}$-Fluoralkyl steht beispielsweise für Trifluormethyl, Pentafluorethyl, Heptafluorpropyl oder Perfluorbutyl.

$C_1$-$C_{12}$-Hydroxyalkyl steht für einen $C_1$-$C_{12}$-Alkylrest gemäß vorstehender Definition, der einfach oder mehrfach durch Hydroxygruppen substituiert ist.

Beispielsweise steht $C_1$-$C_{12}$-Hydroxyalkyl für Hydroxymethyl, 2-Hydroxyethyl, 2-Hydroxypropyl, 3-Hydroxypropyl.

$C_2$-$C_{12}$-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-buteny, 1-Hexenyl, 1-Heptenyl, 1-Octenyl oder 2-Octenyl.

Die Verbindungen der Formel (I) und ihre Herstellung sind grundsätzlich bekannt und beispielsweise in Ionic Liquids in Synthesis, 2nd Ed., 2007, Wiley-VCH, Weinheim (Hrsg. Wasserscheid und Welton) offenbart.

Erfindungsgemäß werden Diastereomere des 2-Isopropyl-5-methylcyclohexanols aus Stoffgemischen enthaltend zumindest zwei zueinander diastereomere Verbindungen des 2-Isopropyl-5-methylcyclohexanols mittels extraktiver Rektifikation getrennt.

Im Rahmen der Erfindung bedeutet dies dass zumindest ein Stereoisomer ausgewählt aus der Gruppe d- und 1-Menthol, d- und 1-Neomenthol, d- und 1-Isomenthol und d- und 1-Neoisomenthol von zumindest einem anderen dazu Diastereomeren Isomer ausgewählt aus der gleichen vorgenannten Gruppe getrennt wird.

So kann beispielsweise

- 1-Menthol oder d-Menthol bzw. eine beliebige Mischung von d- und 1-Menthol von einer beliebigen Mischung enthaltend eine, zwei, drei, vier, fünf oder alle dazu diastereomeren Verbindungen aus der Gruppe d- und 1-Neomenthol, d- und 1-Isomenthol und d- und 1-Neoisomenthol

- d- oder 1- Isomenthol bzw. eine beliebige Mischung von d- und 1-Isomenthol von einer beliebigen Mischung enthaltend eine, zwei, drei, vier, fünf oder alle dazu diastereomeren Verbindungen aus der Gruppe 1-Menthol und d-Menthol, d- und 1-Neomenthol, und d- und 1-Neoisomenthol

- d- oder 1- Neomenthol bzw. eine beliebige Mischung von d- und 1-Neomenthol von einer beliebigen Mischung enthaltend eine, zwei, drei, vier, fünf oder alle dazu diastereomeren Verbindungen aus der Gruppe 1-Menthol und d-Menthol, d- und 1-Isomenthol, und d- und 1-Neoisomenthol

- d- oder 1- Neoisomenthol bzw. eine beliebige Mischung von d- und 1-Neoisomenthol von einer beliebigen Mischung enthaltend eine, zwei, drei, vier, fünf oder alle dazu diastereomeren Verbindungen aus der Gruppe 1-Menthol und d-Menthol, d- und 1-Isomenthol, und d- und 1-Neomenthol

abgetrennt werden.

Beliebige Mischungen von d- und 1-Stereoisomeren umfassen dabei auch einzelne Racemate oder beliebige Mischungen von Racematen.

Vorzugsweise wird
1-Menthol oder d-Menthol bzw. eine beliebige Mischung von d- und 1-Menthol von einer beliebigen Mischung enthaltend eine, zwei, drei, vier, fünf oder alle dazu diastereomeren Verbindungen aus der Gruppe d- und 1-Neomenthol, d- und 1- Isomenthol und d- und 1-Neoisomenthol abgetrennt wobei vorzugsweise racemische Mischungen von d- und 1-Menthol von einer Mischung enthaltend eine, zwei oder alle racemischen Mischungen aus der Gruppe d, 1-Neomenthol, d,1- Isomenthol und d,1-Neoisomenthol abgetrennt werden.

Das jeweilige Verhältnis der zu trennenden Diastereomeren im eingesetzten Stoffgemisch ist dabei prinzipiell nicht begrenzt.

In einer Ausführungsform wird die Rektifikation so ausgeführt, dass ein Stoffgemisch enthaltend im Wesentlichen die zu trennenden Diastereomere des 2-Isopropyl-5-methylcyclohexanols der Rektifikation unterworfen wird, wobei der Begriff "im Wesentlichen" hierbei bedeuten soll , dass der Anteil sonstiger Komponenten über die zu trennenden Diastereomere des 2-Isopropyl-5-methylcyclohexanols hinaus entweder 0 oder mehr als 0 bis 30, bevorzugt mehr als 0 bis 10 Gew.-% und besonders bevorzugt mehr als 0 bis 2 Gew.-% an weiteren Verbindungen enthalten kann.

Geeignete Stoffgemische können beispielsweise solche sein die direkt bei der Hydrierung von Thymol als Reaktionsgemisch gewonnen werden oder nach einer ersten Destillation des vorgenannten Reaktionsgemisches z.B.

als Kopf- Sumpf- oder Seitenstrom erhalten werden.

In diesen Fällen sind die vorgenannten weiteren Verbindungen zum Beispiel nicht umgesetztes Thymol und/oder Nebenprodukte aus der Hydrierung von Thymol.

In einer bevorzugten Ausführungsform werden daher Stoffgemische in das erfindungsgemäße Verfahren eingesetzt, die

- 60 bis 90 Gew.-%, vorzugsweise 70 bis 85 Gew.-% Menthol (d.h. d-Menthol, 1-Menthol oder beliebige Mischungen davon einschließlich des Racemates) und
- 5 bis 35 Gew.-%, vorzugsweise 12 bis 30 Gew.-% Isomenthol (d.h. d-Isomenthol, 1-Isomenthol oder beliebige Mischungen davon einschließlich des Racemates) und
- entweder keine oder mehr als 0 bis 5 Gew.- an den weiteren 4 Stereoisomeren des 2-Isopropyl-5-methylcyclohexanols enthalten, wobei

die Summe der vorgenannten Stereoisomere des 2-Isopropyl-5-methylcyclohexanols im Stoffgemisch 97 Gew.-% oder mehr, vorzugsweise 98 Gew.-% oder mehr und besonders bevorzugt 99 Gew.-% oder mehr beträgt.

Zur Rektifikation können alle dem Fachmann bekannten, zur Rektifikation geeigneten Vorrichtungen wie insbesondere Kolonnen eingesetzt werden. Solche Kolonnen umfassen einfache Kolonnen mit oder ohne Einbauten, Seitenstromkolonnen, Trennwandkolonnen oder thermisch gekoppelte Destillationskolonnen.

Vorzugsweise wird das in die Extraktivrektifikation eingesetzte Stoffgemisch zunächst auf eine Temperatur gebracht oder weist diese auf, die in Abhängigkeit vom gewählten Druck bei der Rektifikation zwischen 20 und 180°C und vorzugsweise zwischen 80 und 160°C liegt. In einer bevorzugten Ausführungsform liegt die Sumpftemperatur in der Rektifikationskolonne bei 180°C oder weniger, besonders bevorzugt bei 160°C oder weniger.

In einer bevorzugten Ausführungsform beträgt der Druck am Kolonnenkopf im allgemeinen und abhängig vom zu trennenden Gemisch beispielsweise im Bereich von 5 bis 500 hPa, bevorzugt bei 5 bis 200 hPa.

Die Druckdifferenz zwischen Kolonnensumpf und Kolonnenkopf kann beispielsweise 0 bis 200, bevorzugt 1 bis 100 hPa betragen.

Die Anzahl der theoretischer Trennstufen kann beispielsweise 5 bis 1000, vorzugsweise 30 bis 300 betragen.

Das Gewichtsverhältnis von Rücklauf zu Destillatentnahme kann beispielsweise 0,5:1 bis 20:1, besonders bevorzugt 2:1 bis 8:1 betragen.

Das Gewichtsverhältnis von ionischen Flüssigkeiten bzw. der Verbindungen der Formeln (I) zur Menge des zu trennenden Stoffgemisches, das der Rektifikationskolonne zugeführt wird, kann beispielsweise 2:1 bis 40:1, bevorzugt 2:1 bis 8:1 betragen.

Das erfindungsgemäße Verfahren kann batchweise oder kontinuierlich, vorzugsweise kontinuierlich durchgeführt werden.

Insbesondere bei kontinuierlicher Fahrweise kann die Zuführung des zu trennenden Stoffgemisches und der ionischen Flüssigkeit in an sich bekannter Weise erfolgen, vorzugsweise erfolgt die Einspeisung der ionischen Flüssigkeit in die Kolonne oberhalb der Einspeisung des zu trennenden Stoffgemisches.

Bei der Rektifikation werden typischerweise mindestens zwei Produktströme erhalten, wobei beispielsweise ein Diastereomer oder Diastereomerenpaar des 2-Isopropyl-5-methylcyclohexanols als Produktstrom über Kopf oder im Seitenstrom entnommen werden kann und das oder die weiteren Diastereomere oder Diastereomerenpaare mit der oder den ionischen Flüssigkeiten als Sumpfstrom.

In einer bevorzugten Ausführungsform des erfindungsgemäßen Verfahrens wird die ionische Flüssigkeit wieder gewonnen und erneut zur Trennung eingesetzt.

Zur Rückgewinnung der ionischen Flüssigkeiten bzw. der Verbindungen der Formel (I) erfolgt zum Beispiel durch destillative Abtrennung aus dem Sumpfstrom. Vorzugsweise erfolgt die destillative Abtrennung in zwei Trennstufen ohne Rücklauf Dabei wird in der ersten Trennstufe bei einem Druck im Bereich von 5 bis 200 hPa und bevorzugt 5 bis 50 hPa das oder die im Sumpfstrom enthaltenen Diastereomere oder Diastereomerenpaare des 2-Isopropyl-5-methylcyclohexanols weitestgehend abgetrennt, wobei unter dem Begriff "weitestgehend" hierbei eine mehr als 90%-ige Abtrennung, vorzugsweise eine mehr als 95%-ige Abtrennung zu verstehen ist. In einer zweiten Trennstufe erfolgt danach eine weitere Abtrennung bei einem niedrigeren Druck als in der ersten Trennstufe von beispielsweise 1 bis 20 hPa und bevorzugt 1 bis 5 hPa. Die Kondensation von Produktbrüden findet zweckmäßigerweise nach einer Druckanhebung auf beispielsweise 5 bis 200 hPa und bevorzugt 5 bis 50 hPa statt.

Der besondere Vorteil des erfindungsgemäßen Verfahrens ist, dass in effizienter Weise Mischungen von diastereomeren 2-Isopropyl-5-methylcyclohexanolen derart getrennt werden können, dass Diastereomere oder Diastereomerenpaare mit hohen Reinheiten erhalten werden können.

Weiterer Gegenstand der Erfindung ist daher die Verwendung von ionischen Flüssigkeiten, insbesondere von

Verbindungen der Formel (I) zur Trennung von Diastereomeren des 2-Isopropyl-5-methylcyclohexanols.

Erfindungsgemäß kann der spezifische Energieverbrauch deutlich gesenkt und die Abmaße der verwendeten Trennapparate, d.h. das notwendige Apparatevolumen je erforderlicher Trennstufe, erheblich reduziert werden.

## Beispiele

### Trennung von Diastereomerengemischen

Folgende Koeffizienten von Aktivitätsfaktoren wurden mit Hilfe des Programms COSMO-RS der Fa.COSMOlogic GmbH & Co KG unter der Annahme unendlicher Verdünnung und einer Temperatur von 130°C ermittelt:

| Entrainer | $\gamma$.(Menthol)/$\gamma$(Isomenthol) |
|---|---|
| 1,3-Dihydroxyimidazolium-hexafluorophosphat | 1,25 |
| Guanidinium-bis(trifluoromethylsulfonyl)amid | 1,19 |
| 2-Hydroxyethylammoniumbis(trifluoromethylsulfonyl)amid | 1,19 |
| Imidazolium-bis(trifluoromethylsulfonyl)amid | 1,18 |
| Butylammonium-triflat | 1,17 |
| Trimethylammonium-methansulfonat | 1,17 |
| 1,3-Dihydroxyimidazolium-bis(trifluoromethylsulfonyl)amid | 1,17 |
| N,N-Dimethylethanolammonium-bis(trifluoromethylsulfonyl)amid | 1,16 |
| 1,3-Dihydroxy-2-methylimidazolium-bis(trifluoromethylsulfonyl)amid | 1,15 |
| 1-Butylimidazolium-hexafluorophosphat | 1,15 |
| Guanidinium-tris(pentafluoroethyl)trifluorophosphat | 1,15 |

Die Prüfungen zeigten, dass das erfindungsgemäße Verfahren im Vergleich zum Stand der Technik deutlich überlegen sind.

### Destillationsbeispiel

Eine Extraktionskolonne mit 220 theoretischen Trennstufen wurde mit dem zu trennenden Stoffgemisch bestehend aus 84 Gew.-% d,1-Menthol und 16 Gew.-% d,1-Isomenthol mit einer Temperatur von 140°C und dem Extraktionsmittel 1,3-Dihydroxyimidazolium-hexafluorophosphat mit einer Temperatur von 140°C gespeist. Dabei wurde der Entrainer oberhalb des vorgenannten Stoffgemisches in die Kolonne eingespeist. Das Verhältnis von Menthol zu Entrainer lag bei 1:8.

Der Druck am Kolonnenkopf betrug 80 hPa und im Kolonnensumpf 160 hPa. Das Extraktionsmittel verließ den Kolonnensumpf mit einer Temperatur von etwa 150°C. Im Sumpfstrom waren mehr als 99 Gew.-% des eingesetzten Isomenthols und weniger als 1 Gew.-% des eingesetzten Menthols enthalten. Im Kopfstrom der Kolonne wurde hochreines Menthol mit einem Gehalt von mehr als 99 Gew.-% entnommen, wobei das Rücklaufverhältnis (Rückflussmenge/Destillat) bei 4:1 lag.

## Patentansprüche

1. Verfahren zur Trennung von Diastereomeren des 2-Isopropyl-5-methylcyclohexanols aus Stoffgemischen enthaltend zumindest zwei zueinander diastereomere Verbindungen des 2-Isopropyl-5-methylcyclohexanols mittels extraktiver Rektifikation, das **dadurch gekennzeichnet ist, dass** die Rektifikation in Gegenwart einer oder mehrerer ionischer Flüssigkeiten durchgeführt wird.

2. Verfahren nach Anspruch 1, **dadurch gekennzeichnet, dass** als ionische Flüssigkeiten solche der Formel (I) eingesetzt werden

$$[K^+] [A^-] \qquad (I)$$

in der
$[K^+]$ für ein Kation steht, das ausgewählt ist aus der Gruppe der Formeln (IIa) bis (IIe)

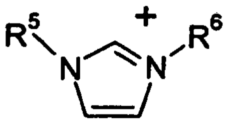 (IIa) 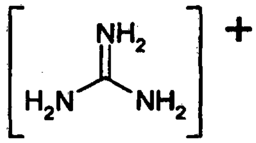 (IIb) 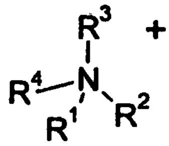 (IIc)

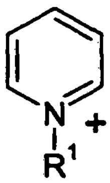 (IId) 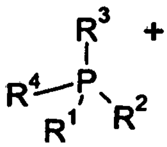 (IIe)

und
$[A^-]$ für ein Anion steht, das ausgewählt ist aus der Gruppe:
Hexafluorophosphat, Tetrafluoroborat, Chlorid, Bromid, Iodid, Bis(trifluormethylsulfonyl)amid und solchen der Formeln (IIIa), (IIIb) und (IIIc)

| | |
|---|---|
| (IIIa) | $R^7\text{-}SO_3^-$ |
| (IIIb) | $^-O(P{=}O)(OR^3)(OR^4)$ |
| (IIIc) | $^-O(P{=}O)(R^3)(OR^4)$ |

wobei in den Formeln (IIa) bis (IIe) sowie den Formeln (IIIa) bis (IIIc)

• die Reste $R^1$, $R^2$, $R^3$ und $R^4$ jeweils völlig unabhängig voneinander für Wasserstoff, $C_1$-$C_{12}$-Alkyl, $C_1$-$C_{12}$-Fluoralkyl, $C_2$-$C_{12}$-Alkylen, oder $C_1$-$C_{12}$-Hydroxyalkyl stehen, wobei $C_1$-$C_{12}$-Alkyl, beziehungsweise $C_2$-$C_{12}$-Alkylen, beziehungsweise $C_2$-$C_{12}$-Alkenylen jeweils unabhängig einen geradkettigen, ganz oder teilweise cyclischen, verzweigten oder unverzweigten Alkylbeziehungsweise Alkylen- beziehungsweise Alkenylen-Rest mit der angegebenen Zahl an Kohlenstoffatomen entspricht und wobei die genannten Reste nicht, einfach oder mehrfach, bevorzugt nicht oder einfach weiterhin durch $C_1$-$C_4$-Alkoxyreste oder Halogene substituiert sein können, oder
zwei Reste zusammen für $C_2$-$C_{12}$-Alkenylen stehen
wobei für die Formeln (IIc) und (IIe) die Maßgabe gilt dass maximal zwei der Reste $R^1$, $R^2$, $R^3$ und $R^4$ für Wasserstoff stehen
und
• die Reste $R^5$ und $R^6$ jeweils völlig unabhängig voneinander für Wasserstoff, Hydroxy, $C_1$-$C_{12}$-Alkyl, $C_1$-$C_{12}$-Fluoralkyl oder $C_1$-$C_{12}$-Hydroxyalkyl stehen
und
• der Rest $R^7$ für einen Phenyl-Rest steht, der nicht, einfach oder mehrfach durch Reste substituiert ist die ausgewählt sind aus der Gruppe $C_1$-$C_{12}$-Alkyl, $C_1$-$C_{12}$-Fluoralkyl, Fluor, Chlor, Brom, Hydroxy oder $C_1$-$C_{12}$-Hydroxyalkyl.

3. Verfahren nach Anspruch 2, **dadurch gekennzeichnet, dass** als ionische Flüssigkeiten solche der Formel (I) eingesetzt werden, in denen $[K^+]$ für ein Kation der Formel (IIa) oder (IIb) steht und $[A^-]$ für Hexafluorophosphat oder Bis(trifluormethylsulfonyl)amid.

4. Verfahren nach einem der Ansprüche 1 bis 3, **dadurch gekennzeichnet, dass** als ionische Flüssigkeiten 1,3-Dihydroxyimidazolium-hexafluorophosphat, Guanidinium-bis(trifluoromethylsulfonyl)-amid, 2-Hydroxyethylammoniumbis(trifluoromethylsulfonyl)amid, Imidazolium-bis(trifluoromethylsulfonyl)amid, Butylammonium-triflat, Trimethylammonium-methansulfonat, 1,3-Dihydroxyimidazolium-bis(trifluoromethylsulfonyl)amid, N,N-Dimethylethanolammonium-bis(trifluoromethylsulfonyl)amid, 1,3-Dihydroxy-2-methyl-imidazolium-bis(trifluoromethylsulfonyl)amid, 1-Butylimidazoliumhexafluorophosphat und Guanidinium-tris(pentafluoroethyl)trifluorophosphat oder beliebige Mischungen der vorgenannten Verbindungen eingesetzt werden.

**5.** Verfahren nach einem der Ansprüche 1 bis 4, **dadurch gekennzeichnet, dass**

• 1-Menthol oder d-Menthol bzw. eine beliebige Mischung von d- und 1-Menthol von einer beliebigen Mischung enthaltend eine, zwei, drei, vier, fünf oder alle dazu diastereomeren Verbindungen aus der Gruppe d- und 1-Neomenthol, d- und 1- Isomenthol und d- und l-Neoisomenthol
• d- oder 1- Isomenthol bzw. eine beliebige Mischung von d- und 1-Isomenthol von einer beliebigen Mischung enthaltend eine, zwei, drei, vier, fünf oder alle dazu diastereomeren Verbindungen aus der Gruppe 1-Menthol und d-Menthol, d- und l-Neomenthol, und d- und 1-Neoisomenthol
• d- oder 1- Neomenthol bzw. eine beliebige Mischung von d- und 1-Neomenthol von einer beliebigen Mischung enthaltend eine, zwei, drei, vier, fünf oder alle dazu diastereomeren Verbindungen aus der Gruppe 1-Menthol und d-Menthol, d- und 1-Isomenthol, und d- und 1-Neoisomenthol
• d- oder 1- Neoisomenthol bzw. eine beliebige Mischung von d- und 1-Neoisomenthol von einer beliebigen Mischung enthaltend eine, zwei, drei, vier, fünf oder alle dazu diastereomeren Verbindungen aus der Gruppe 1-Menthol und d-Menthol, d- und 1-Isomenthol, und d- und 1-Neomenthol
voneinander getrennt werden.

**6.** Verfahren nach einem der Ansprüche 1 bis 5, **dadurch gekennzeichnet, dass** 1-Menthol oder d-Menthol bzw. eine beliebige Mischung von d- und 1-Menthol von einer beliebigen Mischung enthaltend eine, zwei, drei, vier, fünf oder alle dazu diastereomeren Verbindungen aus der Gruppe d- und 1-Neomenthol, d- und 1-Isomenthol und d- und 1-Neoisomenthol abgetrennt werden.

**7.** Verfahren nach einem der Ansprüche 1 bis 6, **dadurch gekennzeichnet, dass** als Stoffgemische solche eingesetzt werden, die direkt bei der Hydrierung von Thymol als Reaktionsgemisch gewonnen werden oder nach einer ersten Destillation des vorgenannten Reaktionsgemisches erhalten werden.

**8.** Verfahren nach einem der Ansprüche 1 bis 7, **dadurch gekennzeichnet, dass** Stoffgemische eingesetzt werden, die

• 60 bis 90 Gew.-%, vorzugsweise 70 bis 85 Gew.-% Menthol (d.h. d-Menthol, 1-Menthol oder beliebige Mischungen davon einschließlich des Racemates) und
• 5 bis 35 Gew.-%, vorzugsweise 12 bis 30 Gew.-% Isomenthol (d.h. d-Isomenthol, l-Isomenthol oder beliebige Mischungen davon einschließlich des Racemates) und
• entweder keine oder mehr als 0 bis 5 Gew.- an den weiteren 4 Stereoisomeren des 2-Isopropyl-5-methylcyclohexanols enthalten,
wobei die Summe der vorgenannten Stereoisomere des 2-Isopropyl-5-methylcyclohexanols im Stoffgemisch 97 Gew.-% oder mehr, vorzugsweise 98 Gew.-% oder mehr und besonders bevorzugt 99 Gew.-% oder mehr beträgt.

**9.** Verfahren nach einem der Ansprüche 1 bis 8, **dadurch gekennzeichnet, dass** das Verfahren in einer Rektifikationskolonne durchgeführt wird und das Gewichtsverhältnis von Rücklauf zu Destillatentnahme 0,5:1 bis 20:1, besonders bevorzugt 2:1 bis 8:1 beträgt.

**10.** Verfahren nach einem der Ansprüche 1 bis 9, **dadurch gekennzeichnet, dass** das Gewichtsverhältnis von ionischen Flüssigkeiten zur Menge des zu trennenden Stoffgemisches beispielsweise 2:1 bis 40:1, bevorzugt 2:1 bis 8:1 beträgt.

**11.** Verfahren nach einem der Ansprüche 1 bis 10, **dadurch gekennzeichnet, dass** die ionische Flüssigkeit wieder gewonnen und erneut zur Trennung eingesetzt wird.

**12.** Verfahren nach einem der Ansprüche 1 bis 11, **dadurch gekennzeichnet, dass** es kontinuierlich durchgeführt wird.

**13.** Verfahren nach einem Ansprüche 1 bis 12, **dadurch gekennzeichnet, dass** das Verfahren in einer Rektifikationskolonne durchgeführt wird und die Zuführung des zu trennenden Stoffgemisches derart erfolgt, dass die Einspeisung der ionischen Flüssigkeit in die Kolonne oberhalb der Einspeisung des zu trennenden Stoffgemisches erfolgt.

**14.** Verwendung von ionischen Flüssigkeiten, vorzugsweise von solchen gemäß Anspruch 2, 3 oder 4 zur Trennung von Diastereomeren des 2-Isopropyl-5-methylcyclohexanols.

## Claims

**1.** Process for separating diastereomers of 2-isopropyl-5-methylcyclohexanol from compositions of matter containing at least two mutually diastereomeric compounds of 2-isopropyl-5-methylcyclohexanol via extractive rectification, said process being **characterized in that** the rectification is performed in the presence of one or more ionic liquids.

**2.** Process according to Claim 1, **characterized in that** ionic liquids used are of formula (I)

$[K^+] [A^-]$ (I)

where

$[K^+]$ represents a cation selected from the group of formulae (IIa) to (IIe)

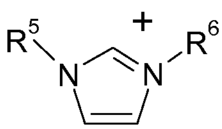
(IIa)
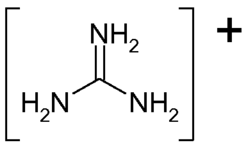
(IIb)
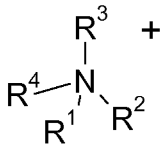
(IIc)

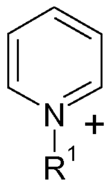
(IId)
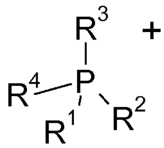
(IIe)

and
$[A^-]$ represents an anion selected from the group:

hexafluorophosphate, tetrafluoroborate, chloride, bromide, iodide, bis(trifluoromethylsulphonyl)amide and those of formulae (IIIa), (IIIb) and (IIIc)

(IIIa) $R^7-SO_3^-$

(IIIb) $-O(P=O)(OR^3)(OR^4)$

(IIIc) $-O(P=O)(R^3)(OR^4)$

where, in the formulae (IIa) to (IIe) and also the formulae (IIIa) to (IIIc),

• the radicals $R^1$, $R^2$, $R^3$ and $R^4$ each fully independently represent hydrogen, $C_1$-$C_{12}$-alkyl, $C_1$-$C_{12}$-fluoroalkyl, $C_2$-$C_{12}$-alkylene or $C_1$-$C_{12}$-hydroxyalkyl, where $C_1$-$C_{12}$-alkyl, $C_2$-$C_{12}$-alkylene and $C_2$-$C_{12}$-alkenylene each independently correspond respectively to a straight-chain, wholly or partly cyclic, branched or unbranched alkyl, alkylene or alkenylene radical having the stated number of carbon atoms and where the radicals mentioned may have no, single or multiple, preferably no or single, further substitution by $C_1$-$C_4$-alkoxy radicals or halogens, or
two radicals combine to represent $C_2$-$C_{12}$-alkenylene,
where the formulae (IIc) and (IIe) are subject to the proviso that not more than two of $R^1$, $R^2$, $R^3$ and $R^4$ represent hydrogen,
and

• the radicals $R^5$ and $R^6$ each fully independently represent hydrogen, hydroxyl, $C_1$-$C_{12}$-alkyl, $C_1$-$C_{12}$-fluoroalkyl or $C_1$-$C_{12}$-hydroxyalkyl,
and
• the radical $R^7$ represents a phenyl radical with no, single or multiple substitution by radicals selected from the group $C_1$-$C_{12}$-alkyl, $C_1$-$C_{12}$-fluoroalkyl, fluorine, chlorine, bromine, hydroxyl or $C_1$-$C_{12}$-hydroxyalkyl.

**3.** Process according to Claim 2, **characterized in that** ionic liquids used are of formula (I) where $[K^+]$ represents a cation of formula (IIa) or (IIb) and $[A^-]$ represents hexafluorophosphate or bis(trifluoromethylsulphonyl)amide.

**4.** Process according to any of Claims 1 to 3, **characterized in that** ionic liquids used comprise 1,3-dihydroxyimidazolium hexafluorophosphate, guanidinium bis(trifluoromethylsulphonyl)amide, 2-hydroxyethylammonium bis(trifluoromethylsulphonyl)amide, imidazolium bis(trifluoromethylsulphonyl)amide, butylammonium triflate, trimethylammonium methansulphonate, 1,3-dihydroxyimidazolium bis(trifluoromethylsulphonyl)amide, N,N-dimethylethanolammonium bis(trifluoromethylsulphonyl)amide, 1,3-dihydroxy-2-methyl-imidazolium-bis(trifluoromethylsulphonyl)amide, l-butylimidazolium hexafluorophosphate and guanidinium tris(pentafluoroethyl)trifluorophosphate or any desired mixtures thereof.

**5.** Process according to any of Claims 1 to 4, **characterized in that** there are separated from each other

• 1-menthol or d-menthol or any desired mixture of d- and 1-menthol from any desired mixture containing one, two, three, four, five or all relatively diastereomeric compounds from the group d- and l-neomenthol, d- and l-isomenthol and d- and l-neoisomenthol;
• l-isomenthol or d-isomenthol or any desired mixture of d- and l-isomenthol from any desired mixture containing one, two, three, four, five or all relatively diastereomeric compounds from the group 1-menthol and d-menthol, d- and l-neomenthol, and d- and l-neoisomenthol;
• 1-neomenthol or d-neomenthol or any desired mixture of d- and 1-neomenthol from any desired mixture containing one, two, three, four, five or all relatively diastereomeric compounds from the group 1-menthol and d-menthol, d- and l-isomenthol, and d- and 1-neoisomenthol
• l-neoisomenthol or d-neoisomenthol or any desired mixture of d- and 1-neoisomenthol from any desired mixture containing one, two, three, four, five or all relatively diastereomeric compounds from the group l-menthol and d-menthol, d- and l-isomenthol, and d- and 1-neomenthol.

**6.** Process according to any of Claims 1 to 5, **characterized in that** 1-menthol or d-menthol or any desired mixture of d- and l-menthol are separated from any desired mixture containing one, two, three, four, five or all relatively diastereomeric compounds from the group d- and l-neomenthol, d- and 1- isomenthol and d- and l-neoisomenthol.

**7.** Process according to any of Claims 1 to 6, **characterized in that** compositions of matter used are obtained directly as a reaction mixture in the hydrogenation of thymol or following a first distillation of the aforementioned reaction mixture.

**8.** Process according to any of Claims 1 to 7, **characterized in that** compositions of matter used contain

• 60% to 90% by weight and preferably 70% to 85% by weight of menthol (i.e. d-menthol, 1-menthol or any desired mixtures thereof including the racemate) and
• 5% to 35% by weight and preferably 12% to 30% by weight of isomenthol (i.e. d-isomenthol, l-isomenthol or any desired mixtures thereof including the racemate) and
• either none or more than 0% to 5% by weight of the further 4 stereoisomers of 2-isopropyl-5-methylcyclohexanol, wherein the sum total of the aforementioned stereoisomers of 2-isopropyl-5-methylcyclohexanol in the composition of matter is 97% by weight or more, preferably 98% by weight or more and more preferably 99% by weight or more.

**9.** Process according to any of Claims 1 to 8, **characterized in that** the process is carried out in a rectification column and the weight ratio of runback to distillate taken off is in the range from 0.5:1 to 20:1 and more preferably in the range from 2:1 to 8:1.

**10.** Process according to any of Claims 1 to 9, **characterized in that** the weight ratio of ionic liquids to the amount of composition of matter to be separated is for example in the range from 2:1 to 40:1 and preferably in the range from

2:1 to 8:1.

**11.** Process according to any of Claims 1 to 10, **characterized in that** the ionic liquid is recovered and reused for separation.

**12.** Process according to any of Claims 1 to 11, **characterized in that** it is carried out continuously.

**13.** Process according to any of Claims 1 to 12, **characterized in that** the process is carried out in a rectification column and the composition of matter to be separated is fed such that the ionic liquid is fed into the column above the point of feed for the composition of matter to be separated.

**14.** Use of ionic liquids, preferably ionic liquids according to Claim 2, 3 or 4, for separating diastereomers of 2-isopropyl-5-methylcyclohexanol.

**Revendications**

**1.** Procédé pour la séparation de diastéréoisomères du 2-isopropyl-5-méthylcyclohexanol à partir de mélanges de substances contenant au moins deux composés, diastéréoisomères l'un par rapport à l'autre, du 2-isopropyl-5-méthylcyclohexanol, par rectification extractive, qui est **caractérisé en ce qu'**on effectue la rectification en présence d'un ou de plusieurs liquides ioniques.

**2.** Procédé selon la revendication 1, **caractérisé en ce qu'**on utilise comme liquides ioniques ceux de formule (I)

$[K^+][A^-]$ (I)

dans laquelle

$[K^+]$ représente un cation, qui est choisi dans le groupe des formules (IIa) à (IIe)

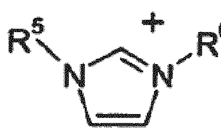
(IIa)

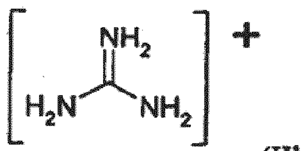
(IIb)

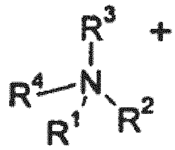
(IIc)

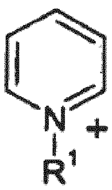
(IId)

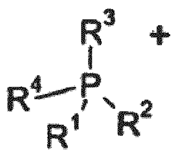
(IIe)

et
$[A^-]$ représente un anion, qui est choisi dans le groupe :

hexafluorophosphate, tétrafluoroborate, chlorure, bromure, iodure, bis(trifluorométhylsulfonyl)amidure et ceux de formules (IIIa), (IIIb) et (IIIc)

(IIIa) $R^7\text{-}SO_3^-$

(IIIb) $-O(P{=}O)(OR^3)(OR^4)$

(IIIc) $O(P{=}O)(R^3)(OR^4)$

dans les formules (IIa) à (IIe) ainsi que dans les formules (IIIa) à (IIIc)

• les radicaux $R^1$, $R^2$, $R^3$ et $R^4$ représentant chacun, totalement indépendamment les uns des autres, un atome d'hydrogène, un groupe alkyle en $C_1$-$C_{12}$, fluoroalkyle ($C_1$-$C_{12}$), alkylène en $C_2$-$C_{12}$ ou hydroxyalkyle($C_1$-$C_{12}$), le groupe alkyle en $C_1$-$C_{12}$, ou respectivement alkylène en $C_2$-$C_{12}$, ou respectivement alcénylène en $C_2$-$C_{12}$ correspondant chaque fois indépendamment à un radical alkyle ou respectivement alkylène ou respectivement alcénylène à chaîne droite, partiellement ou totalement cyclique, ramifié ou non ramifié, ayant le nombre d'atomes de carbone indiqué et lesdits radicaux pouvant être non substitués, une ou plusieurs fois substitués, de préférence non substitués ou monosubstitués encore par des radicaux alcoxy en $C_1$-$C_4$ ou des atomes d'halogène, ou
deux radicaux représentent ensemble un groupe alcénylène en $C_2$-$C_{12}$
étant entendu que pour les formules (IIc) et (IIe) au maximum deux des radicaux $R^1$, $R^2$, $R^3$ et $R^4$ représentent des atomes d'hydrogène
et
• les radicaux $R^5$ et $R^6$ représentent chacun, totalement indépendamment l'un de l'autre, un atome d'hydrogène, un groupe hydroxy, alkyle en $C_1$-$C_{12}$, fluoroalkyle ($C_1$-$C_{12}$) ou hydroxy-alkyle ($C_1$-$C_{12}$) et
• le radical $R^7$ représente un groupe phényle qui est non substitué ou une ou plusieurs fois substitué par des radicaux qui sont choisis dans le groupe constitué par alkyle en $C_1$-$C_{12}$, fluoroalkyle($C_1$-$C_{12}$), fluoro, chloro, bromo, hydroxy ou hydroxyalkyle($C_1$-$C_{12}$).

3. Procédé selon la revendication 2, **caractérisé en ce qu'**on utilise comme liquides ioniques ceux de formule (I), dans lesquels $[K]^+$ représente un cation de formule (IIa) ou (IIb) et $[A^-]$ représente l'hexafluorophosphate ou le bis(trifluorométhylsulfonyl)amidure.

4. Procédé selon l'une quelconque des revendications 1 à 3, **caractérisé en ce qu'**on utilise comme liquides ioniques l'hexafluorophosphate de 1,3-dihydroxyimidazolium, le bis(trifluorométhylsulfonyl)-amidure de guanidinium, le bis(trifluorométhyl-sulfonyl)amidure de 2-hydroxyéthylammonium, le bis(trifluorométhylsulfonyl)amidure d'imidazolium, le triflate de butylammonium, le méthanesulfonate de triméthylammonium, le bis(trifluorométhylsulfonyl)amidure de 1,3-dihydroxyimidazolium, le bis(trifluoro-méthylsulfonyl)amidure de N,N-diméthyléthanolammonium, le bis(trifluorométhylsulfonyl)amidure de 1,3-dihydroxy-2-méthyl-imidazolium, l'hexafluorophosphate de 1-butylimidazolium et le tris(pentafluoroéthyl)trifluorophosphate de guanidinium ou des mélanges quelconques des composés précités.

5. Procédé selon l'une quelconque des revendications 1 à 4, **caractérisé en ce qu'**on sépare les uns des autres

• du l-menthol ou d-menthol ou un mélange quelconque de d- et 1-menthol d'avec un mélange quelconque contenant un, deux, trois, quatre, cinq ou tous les composés diastéréoisomères de ceux-ci, appartenant au groupe constitué par le d- et le l-néomenthol, le d- et le 1-isomenthol, et le d- et le l-néo-isomenthol
• du d- ou l-isomenthol ou un mélange quelconque de d- et l-isomenthol d'avec un mélange quelconque contenant un, deux, trois, quatre, cinq ou tous les composés diastéréoisomères de ceux-ci, appartenant au groupe constitué par le l-menthol et le d-menthol, le d- et le l-néomenthol, et le d- et le 1-néo-isomenthol
• du d- ou 1-néomenthol ou un mélange quelconque de d- et 1-néomenthol d'avec un mélange quelconque contenant un, deux, trois, quatre, cinq ou tous les composés diastéréoisomères de ceux-ci, appartenant au groupe constitué par le 1-menthol et le d-menthol, le d- et le 1-isomenthol, et le d- et le l-néo-isomenthol
• du d- ou l-néo-isomenthol ou un mélange quelconque de d- et l-néo-isomenthol d'avec un mélange quelconque contenant un, deux, trois, quatre, cinq ou tous les composés diastéréoisomères de ceux-ci, appartenant au groupe constitué par le 1-menthol et le d-menthol, le d- et le 1-isomenthol, et le d- et le l-néomenthol.

6. Procédé selon l'une quelconque des revendications 1 à 5, **caractérisé en ce qu'**on sépare du 1-menthol ou du d-menthol ou un mélange quelconque de d- et 1-menthol d'avec un mélange quelconque contenant un, deux, trois, quatre, cinq ou tous les composés diastéréoisomères de ceux-ci, appartenant au groupe constitué par le d- et le l-néomenthol, le d- et le 1-isomenthol et le d- et le l-néo-isomenthol.

7. Procédé selon l'une quelconque des revendications 1 à 6, **caractérisé en ce qu'**on utilise comme mélanges de substances ceux qui sont obtenus en tant que mélange réactionnel directement dans l'hydrogénation du thymol ou sont obtenus après une première distillation du mélange réactionnel précité.

**8.** Procédé selon l'une quelconque des revendications 1 à 7, **caractérisé en ce qu'**on utilise des mélanges de substances qui contiennent

- 60 à 90 % en poids, de préférence 70 à 85 % en poids de menthol (c'est-à-dire d-menthol, 1-menthol ou des mélanges quelconques de ceux-ci y compris du racémate) et
- 5 à 35 % en poids, de préférence 12 à 30 % en poids d'isomenthol (c'est-à-dire d-isomenthol, 1-isomenthol ou des mélanges quelconques de ceux-ci y compris du racémate) et
- soit aucun soit plus de 0 à 5 % en poids des 4 autres stéréoisomères du 2-isopropyl-5-méthylcyclohexanol, la somme des stéréoisomères précités du 2-isopropyl-5-méthylcyclohexanol dans le mélange de substances valant 97 % en poids ou plus, de préférence 98 % en poids ou plus et de façon particulièrement préférée 99 % en poids ou plus.

**9.** Procédé selon l'une quelconque des revendications 1 à 8, **caractérisé en ce qu'**on effectue le procédé dans une colonne de rectification et le rapport pondéral du reflux au prélèvement de distillat vaut de 0,5:1 à 20:1, de façon particulièrement préférée de 2:1 à 8:1.

**10.** Procédé selon l'une quelconque des revendications 1 à 9, **caractérisé en ce que** le rapport pondéral des liquides ioniques à la quantité du mélange de substances à fractionner vaut par exemple de 2:1 à 40:1, de préférence de 2:1 à 8:1.

**11.** Procédé selon l'une quelconque des revendications 1 à 10, **caractérisé en ce qu'**on récupère le liquide ionique et on l'utilise de nouveau pour la séparation.

**12.** Procédé selon l'une quelconque des revendications 1 à 11, **caractérisé en ce qu'**on l'effectue en continu.

**13.** Procédé selon l'une quelconque des revendications 1 à 12, **caractérisé en ce qu'**on effectue le procédé dans une colonne de rectification et l'introduction du mélange de substances à fractionner s'effectue de manière que l'introduction du liquide ionique dans la colonne s'effectue au-dessus de l'arrivée du mélange de substances à fractionner.

**14.** Utilisation de liquides ioniques, de préférence de ceux selon la revendication 2, 3 ou 4, pour la séparation de diastéréoisomères du 2-isopropyl-5-méthylcyclohexanol

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10136614 A
- DE 10154052 A
- US 4874473 A


### In der Beschreibung aufgeführte Nicht-Patentliteratur


- *Chem. Ing. Tech.,* 2003, vol. 75, 1148-1149
- Ionic Liquids in Synthesis. Wiley-VCH, 2007